# EUROPEAN PATENT SPECIFICATION

(11) **EP 1 265 495 B1**
(45) Date of publication and mention of the grant of the patent: **31.08.2011**
(21) Application number: 01936140.1
(22) Date of filing: 22.03.2001
(51) Int. Cl.: A23J 1/20, A23J 1/22, A23C 1/03, C12N 9/10

(54) **METHOD FOR PREPARING A ROLLER DRIED PROTEIN PREPARATION**
VERFAHREN ZUR HERSTELLUNG VON TROMMELGETROCKNETER PROTEINZUBEREITUNG
PROCEDE DE PREPARATION D'UNE PREPARATION DE PROTEINES SECHEES SUR CYLINDRES

(30) Priority: 23.03.2000 WO PCT/EP00/02618
(43) Date of publication of application: 18.12.2002
(73) Proprietor: FrieslandBrands B.V., 3818 LE Amersfoort (NL)
(72) Inventor: CLARK, David, Callam, NL-5175 XG St. Michielsgestel (NL); SCHMELTER, Tillmann, Gerhard, NL-5467 DW Veghel (NL); VAN DIJK, Johannes, Henricus, NL-5409 TN Odiliapeel (NL)
(74) Representative: Van Someren, Petronella F. H. M.
(86) International application number: PCT/EP2001/003436
(87) International publication number: WO 2001/070042

(56) References cited:
- EP-A2- 0 824 870
- EP-A2- 0 824 870
- EP-A2- 0 963 704
- EP-A2- 0 963 704
- EP-B1- 0 821 881
- EP-B1- 0 821 881
- US-A- 3 720 765
- US-A- 4 016 147
- US-A- 4 133 901
- US-A- 4 138 505
- IMM, J. Y., LIAN, P., AND LEE, C. M.: "Gelation and water-binding properties of transglutaminase-treated skim milk powder" JOURNAL OF FOOD SCIENCE., vol. 65, no. 2, 2000, pages 200-205, XP002153299 INSTITUTE OF FOOD TECHNOLOGISTS. CHICAGO., US ISSN: 0022-1147

## Description

The present invention relates to a method for preparing a dried protein preparation.

The preparation of protein powders can for example be achieved by means of spray drying. Spray drying can be effected with starting solutions with protein contents in the range 5-20% (w/w). Protein solutions are frequently processed and atomised at these concentrations as their viscosity properties (0.1-0.5 Pa.s (at a shear rate of 100 s⁻¹ at 70°C (Physica UDS200 rheometer, DIN double gap Z1 geometry) mean that they are easily handled and pumped through standard processing equipment. Spray drying of solutions at the lower end of this protein concentration range can result in high levels of dusting (dust formation), which can result in reduced yields and increased risk of dust explosions. An additional disadvantage of spray drying powders can be the poor wettability and dispersibility properties of the dried powders, which have a low density and tend to float on the surface of water-based liquids.

Powders produced by roller drying are characterized by higher densities and improved wetting and dispersing properties. However, these protein powders have a less favourable taste profile, and have a darker colour than their spray dried equivalents. These defects can be ascribed to the high protein concentration (around 40%) needed for an efficient roller drying process.

Lowering the solids or protein concentration in the roller drying process to e.g. 20% (like in spray drying) would solve this problem. However, protein solutions in the 5-20% (w/w) concentration range do not lend themselves to roller drying. They become too fluid when applied to steam heated rollers and spread to form too thin a film, which burns onto the roller. The resulting film cannot be removed efficiently from the roller as a continuous film by the knives used for this purpose, but the protein is rather removed as scorched dust. Alternatively, a double (twin) roller dryer type can be used in which the protein solution subject to drying is contained in a "pool" between the two roller drums. However, the long residence time in this pool at elevated temperatures causes similar defects to the protein (browning) as observed in a high solids single drum dryer process. Additionally, under these conditions the formation of lysinoalanine (LAL) may be favored, which is not desirable.

US patent 3 720 765 relates to a protein comprising feed material for ruminants. This feed comprises the reaction product of a protein-containing feed material and an organic acid anhydride capable of cross-linking proteins.

EP 0 821 881 A2 relates to a milk-whey protein-containing powder and processed food comprising such powder. This milk whey protein-containing powder is obtained by a process wherein a solution of a milk whey protein-containing composition is reacted with a transglutaminase and the reaction solution is heated and spray-dried.

It is therefore the object of the present invention to provide a new method of drying proteins, that obviates the above stated drawbacks.

This object is achieved according to the invention by a method comprising roller drying a protein solution having a relatively low protein content of less than 40% (w/wt), wherein the proteins in the protein solution to be rollerdried are crosslinked by means of enzymatic treatment with the enzyme transglutaminase, wherein the protein is casein and/or caseinate. The protein content is preferably 15-25% (w/w), more preferably about 20% (w/w). These protein contents correspond to a viscosity in the range 1-10 Pa.s (double gap, Z1 geometry, shear rate 100 s⁻¹ at 70°C) . It was surprisingly found that protein solutions having such relatively low protein content are still viscous enough after crosslinking to be suitable for roller drying. Roller drying is a much simpler technique than spray drying and requires far less complicated equipment. Therefore, malfunction or disturbance of the equipment is less likely to occur than in the case of spray drying.

In addition, no double drum dryer type is needed, since the solution of this cross-linked protein preparation is now viscous enough to be applied on a single drum dryer. This implies that no extra investment for a special drying technique is needed.

The proteins can be crosslinked by means of enzymatic treatment with the enzyme transglutaminase.

The crosslinking is achieved prior to roller drying by exposing an amount of protein to an amount of crosslinking enzyme during an amount of reaction time. For each protein suitable process conditions can be empirically defined. However, as a general rule the amount of protein is 5-30% (w/w), the amount of crosslinking enzyme is 0.01-20 units per gram protein and the reaction time is 0.5-12 hours.

The specific activity of transglutaminase is determined using the method as described by J.E. Folk and P.W. Cole, (1966), J. Biol. Chem., 241, 5518-5525.

The powder resulting from the method as claimed differs from spray dried protein powders in that it is characterized by a higher density, improved wettability and dispersibility. The powder is therefore also part of this invention.

The powder of the invention can be used as a waterbinding agent, emulsifying agent, viscosity increasing agent, texture improving agent, stabilising agent.

The method of the invention can be performed with casein and or caseinates. It was found that the method is particularly suitable for drying caseinate.

In order to control the drying process, the solution containing the crosslinked protein can be heated to adjust the viscosity temporarily, as is normally done in drying operations. Preferably this heating is to about 120°C after which the solution is applied directly to the rollers heated to approximately the same temperature.

The term "crosslinked protein(s)" as used in this application is intended to mean "at least two protein molecules having at least one covalent bond between them".

The present invention will be further illustrated in the examples that follow and which are in no way intended to limit the invention.

### EXAMPLES

### EXAMPLE 1

### Roller drying a crosslinked Protein solution

A solution of sodium caseinate (20% w/w; viscosity 300 mPa.s at 70°C) is reacted with the enzyme transglutaminase [B.C. 2.3.2.13] using 3 units of enzyme per gram caseinate for 1 hour at a temperature of 50°C. The enzyme is deactivated by a heat treatment (90-120°C for 30 to 2 minutes, respectively). This treatment typically results in an increase in viscosity from 0.1-0.5 Pa.s to 1-10 Pa.s at a shear rate of 100 s⁻¹ at 70°C (double gap Z1 geometry). Viscosity measurements show that the crosslinked caseinate solution has now shear-thinning properties.

The crosslinked caseinate solution that has been treated to deactivate the enzyme activity is applied directly to the roller dryer (capacity 10-30 kg per hour) operating at a steam pressure of 2-3 bar and a roller speed of 10-20 rpm. The roller drier is a pilot machine of the make "GMF", equiped as a single drum drier. The
**Figure 5****:** a microphoto of roller dried, crosslinked sodium caseinate; and
**Figure 6****:** a microphoto of spray dried sodium caseinate.

### EXAMPLES

### EXAMPLE 1

### Roller drying a crosslinked protein solution

A solution of sodium caseinate (20% w/w; viscosity 300 mPa.s at 70°C) is reacted with the enzyme transglutaminase [E.C. 2.3.2.13] using 3 units of enzyme per gram caseinate for 1 hour at a temperature of 50°C. The enzyme is deactivated by a heat treatment (90-120°C for 30 to 2 minutes, respectively). This treatment typically results in an increase in viscosity from 0.1-0.5 Pa.s to 1-10 Pa.s at a shear rate of 100 s⁻¹ at 70°C (double gap Z1 geometry). Viscosity measurements show that the crosslinked caseinate solution has now shear-thinning properties.

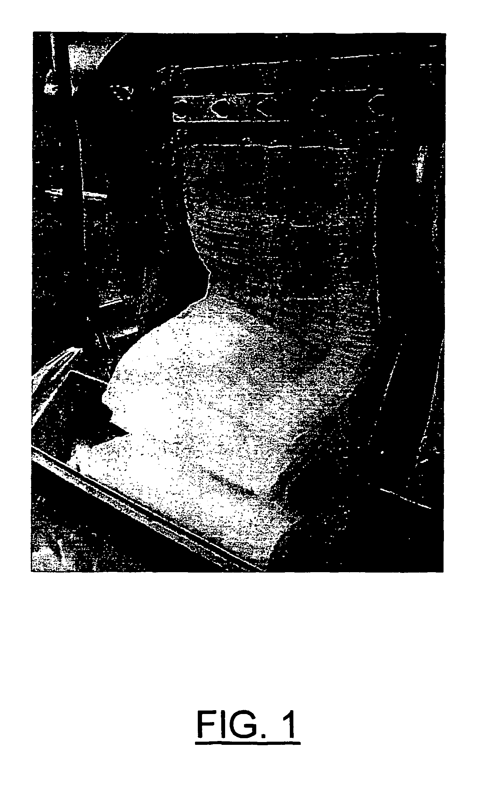

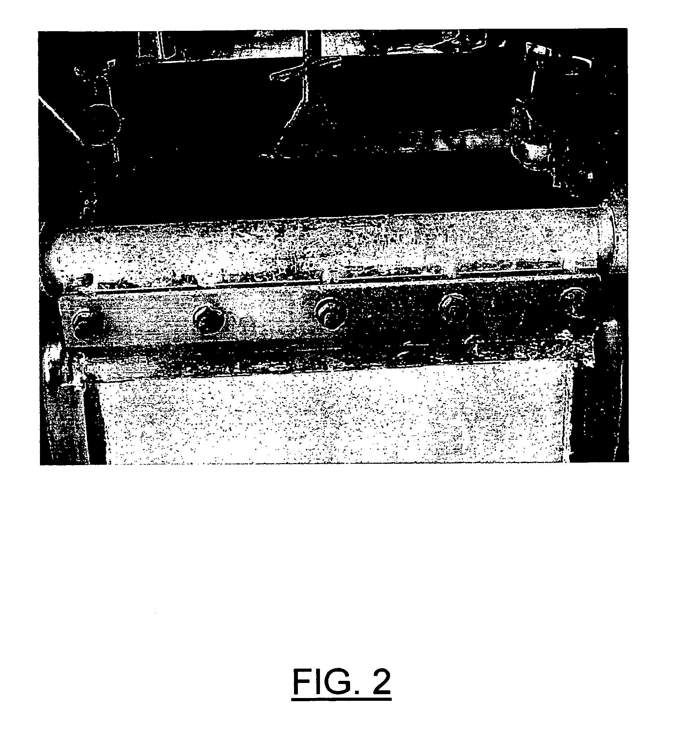

The crosslinked caseinate solution that has been treated to deactivate the enzyme activity is applied directly to the roller dryer (capacity 10-30 kg per hour) operating at a steam pressure of 2-3 bar and a roller speed of 10-20 rpm. The roller drier is a pilot machine of the make "GMF", equiped as a single drum drier. The drum has a diameter of 50 cm, and a length of 50 cm. Despite it's low protein concentration, an excellent protein film was formed on the drum, which could be removed efficiently by the built-in knives (**Figs. 1** **and** **2****)**. The dried film is removed by the built-in knives and fed to a milling machine and sieve.

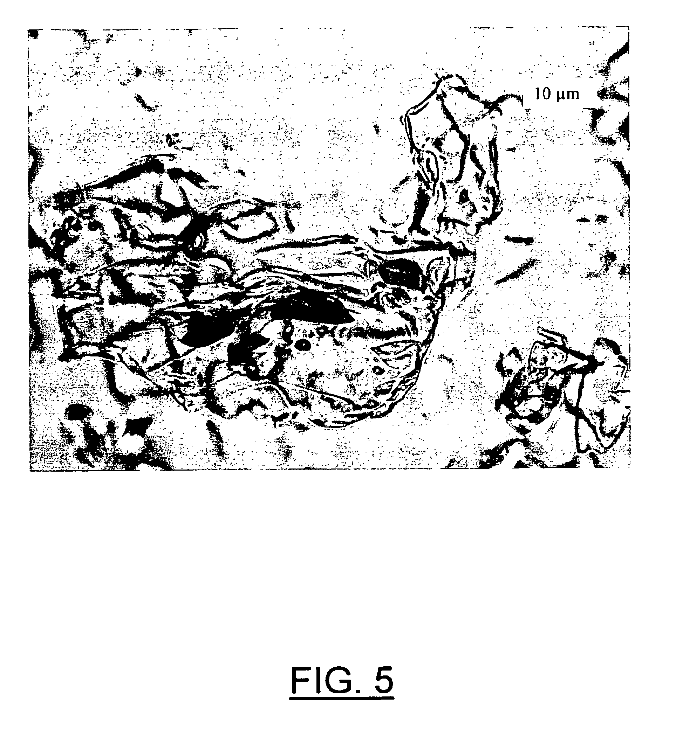

The product thus obtained was redissolved in water to a solids content of 20% (w/w). The viscosity of the solution was measured under the conditions described above and was 8.9 Pa.s. The micrograph of **Fig. 5** (Microscope: Wild M20, dispersion in xylol) shows the microscopic characteristics of a roller dried caseinate product according to the invention, displaying particles of irregular shape, which show frayed ends.

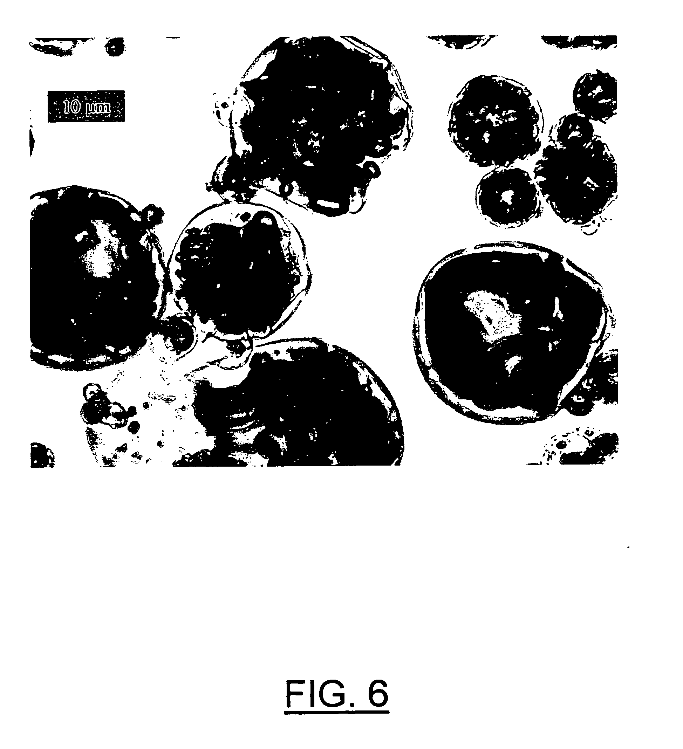

**Fig. 6** shows for comparison a micrograph of spray dried sodium caseinate. The particles are so-called spray globules filled with air pockets.

### EXAMPLE 2

### Comparative example

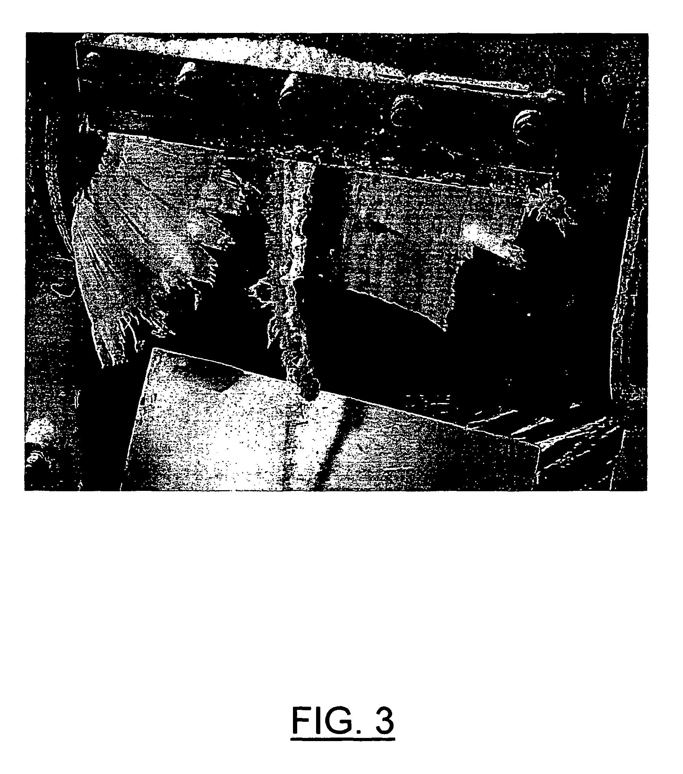

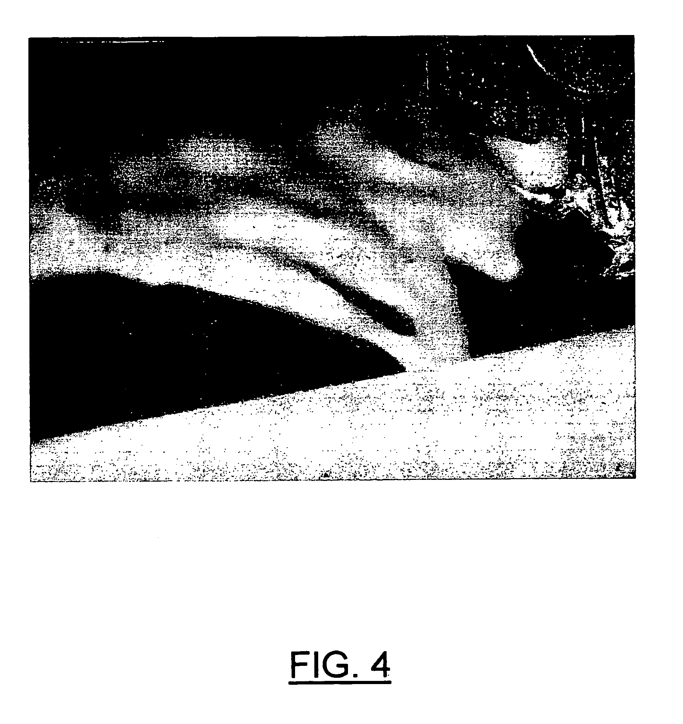

A solution of sodium caseinate (20% w/w; viscosity 300 mPa.s at 70°C, not crosslinked), is applied to the roller drier under the same conditions as in example 1. The solution appears to be too thin to apply to the drum in a good manner **(****Fig. 4****).** A part of the applied feed pours off the drum. The part of the feed solution that remains on the drum results in a very thin, irregular and discontinuous dried film, showing open patches **(****Fig. 3****).** The built in knives could merely remove thin pieces of film and dusty powder.

## Claims

1. Method for roller drying a protein solution having a relatively low protein content of less than 40%, wherein the proteins in the protein solution to be rollerdried are crosslinked by means of enzymatic treatment with the enzyme transglutaminase, wherein the protein is casein and/or caseinate.

2. Method as claimed in claim 1, wherein crosslinking is achieved by exposing an amount of protein to an amount of transglutaminase during an amount of reaction time.

3. Method as claimed in claim 2, wherein the amount of protein is 5-30% (w/w), the amount of transglutaminase is 0.01-20 units per gram protein and the reaction time is 0.5-12 hour.

4. Method as claimed in claims 1-3; wherein the amount of protein is 5-30% (w/w), the amount of transglutaminase is about 0.01 units per gram of protein and the reaction time is 0.5-12 hours.

5. Method as claimed in claim 4, wherein the protein content is 10-25%.

6. Method as claimed in claims 1-5, wherein the protein content is about 20%.

7. Method as claimed in claims 1-6, wherein the viscosity of the protein solution is in the range 1-10 Pa.s at a shear rate of 100 s-1 at 70°C measured at a Physica UDS200 rheometer with DIN double gap Z1 geometry or a comparable device.

8. Protein powder, obtainable by a method as claimed in claims 1-7, wherein the protein is casein and/or caseinate.

9. Protein powder as claimed in claim 8, for use as a waterbinding agent, emulsifying agent, viscosity increasing agent, texture improving agent, stabilising agent.

10. Use of a powder as claimed in claim 8 as a waterbinding agent, emulsifying agent, viscosity increasing agent, texture improving agent, stabilising agent.

11. Use of a powder as claimed in claim 8 as an ingredient in yoghurt or other fermented milk products.

12. Use of a powder as claimed in claim 8 as an ingredient in meat products.

## Patentansprüche

1. Verfahren zur Walzentrocknung einer Proteinlösung mit einem relativ geringen Proteingehalt von weniger als 40%, wobei die Proteine in der Proteinlösung, welche der Walzentrocknung zugeführt werden soll, mittels enzymatischer Behandlung mit dem Enzym Transglutaminase vernetzt werden, wobei das Protein Kasein und/oder Kaseinat ist.

2. Verfahren nach Anspruch 1, wobei die Vernetzung **dadurch** erreicht wird, dass eine Menge an Protein einer Menge an Transglutaminase während einer Dauer an Reaktionszeit ausgesetzt wird.

3. Verfahren nach Anspruch 2, wobei die Menge an Protein 5-30% (w/w), die Menge an Transglutaminase 0,01-20 Einheiten pro Gramm Protein und die Reaktionszeit 0,5-12 Stunden beträgt.

4. Verfahren nach Ansprüchen 1-3, wobei die Menge an Protein 5-30% (w/w), die Menge an Transglutaminase etwa 0,01 Einheiten pro Gramm Protein und die Reaktionszeit 0,5-12 Stunden beträgt.

5. Verfahren nach Anspruch 4, wobei der Proteingehalt 10-25% beträgt.

6. Verfahren nach Ansprüchen 1-5, wobei der Proteingehalt etwa 20% beträgt.

7. Verfahren nach Ansprüchen 1-6, wobei die Viskosität der Proteinlösung im Bereich von 1-10 Pa.s liegt bei einer Schergeschwindigkeit von 100 s⁻¹ bei 70°C, gemessen in einem Physica UDS 200 Rheometer mit Doppelspalt-Geometrie-Z1 nach DIN oder einer vergleichbaren Apparatur.

8. Proteinpulver, erhältlich nach einem Verfahren gemäß Ansprüchen 1-7, wobei das Protein Kasein und/oder Kaseinat ist.

9. Proteinpulver nach Anspruch 8 für die Verwendung als Wasserbindemittel, Emulgator, Mittel zur Erhöhung der Viskosität, Mittel zur Verbesserung der Textur, Stabilisator.

10. Verwendung eines Pulvers nach Anspruch 8 als Wasserbindemittel, Emulgator, Mittel zur Erhöhung der Viskosität, Mittel zur Verbesserung der Textur, Stabilisator.

11. Verwendung eines Pulvers nach Anspruch 8 als ein Bestandteil in Joghurt oder anderen fermentierten Milchprodukten.

12. Verwendung eines Pulvers nach Anspruch 8 als ein Bestandteil in Fleischprodukten.

## Revendications

1. Procédé de séchage sur rouleaux d'une solution protéique ayant une teneur de protéines relativement basse, inférieure à 40 %, dans lequel les protéines dans la solution protéique qui doivent être séchées sur rouleaux sont réticulées par un traitement enzymatique à l'enzyme transglutaminase, la protéine étant la caséine et/ou un caséinate.

2. Procédé selon la revendication 1, dans lequel la réticulation est réalisée par exposition d'une certaine quantité de protéine à une certaine quantité de transglutaminase pendant un certain temps de réaction.

3. Procédé selon la revendication 2, dans lequel la quantité de protéine est de 5 à 30 % (p/p), la quantité de transglutaminase est de 0,01 à 20 unités par gramme de protéine et le temps de réaction est de 0,5 à 12 heures.

4. Procédé selon les revendications 1 à 3, dans lequel la quantité de protéine est de 5 à 30 % (p/p), la quantité de transglutaminase est d'environ 0,01 unité par gramme de protéine et le temps de réaction est de 0,5 à 12 heures.

5. Procédé selon la revendication 4, dans lequel la teneur de protéines est de 10 à 25 %.

6. Procédé selon les revendications 1 à 5, dans lequel la teneur de protéines est d'environ 20 %.

7. Procédé selon les revendications 1 à 6, dans lequel la viscosité de la solution protéique est dans la plage de 1 à 10 Pa.s à un taux de cisaillement de 100 s-1 à 70 °C, mesurée sur un rhéomètre Physica UDS200 ayant la géométrie DIN Z1 double gap ou autre dispositif comparable.

8. Protéines en poudre, pouvant être obtenues par un procédé selon les revendications 1 à 7, dans laquelle la protéine est la caséine et/ou un caséinate.

9. Protéines en poudre selon la revendication 8, utilisables à titre d'agent de rétention d'eau, agent émulsifiant, agent augmentant la viscosité, agent améliorant la texture, agent stabilisant.

10. Utilisation d'une poudre selon la revendication 8 à titre d'agent de rétention d'eau, agent émulsifiant, agent augmentant la viscosité, agent améliorant la texture, 'agent stabilisant.

11. Utilisation d'une poudre selon la revendication 8 à titre d'ingrédient dans un yaourt ou autres produits laitiers fermentés.

12. Utilisation d'une poudre selon la revendication 8 à titre d'ingrédient dans des produits à base de viande.
